# EUROPEAN PATENT SPECIFICATION

(11) **EP 2 328 497 B1**
(45) Date of publication and mention of the grant of the patent: **31.10.2012**
(21) Application number: 09758860.2
(22) Date of filing: 08.04.2009
(51) Int. Cl.: A61B 17/80

(54) **VARIABLE ANGLE FIXATION ELEMENT SYSTEM**
SYSTEM MIT FIXIERELEMENT MIT VARIABLEM WINKEL
SYSTÈME D ÉLÉMENT DE FIXATION À ANGLE VARIABLE

(30) Priority: 03.06.2008 US 58395 P
(43) Date of publication of application: 08.06.2011
(73) Proprietor: Synthes GmbH, 4436 Oberdorf (CH)
(72) Inventor: FRIGG, Robert, CH-2544 Bettlach (CH); HULLIGER, Urs, CH-4543 Deitingen (CH)
(74) Representative: Lusuardi, Werther
(86) International application number: PCT/US2009/039869
(87) International publication number: WO 2009/148697

(56) References cited:
- US-A- 4 029 091
- US-A- 5 690 631
- US-A1- 2004 220 570
- US-A1- 2007 185 489
- US-A1- 2008 058 817

## Description

### Background

Fragments of bones damaged through, for example, fractures, dislocations, degenerative diseases, tumors, etc., are often secured to one another using any of a variety of internal fixation plating systems. Such systems often include a plate attached to the bone or bone portions spanning a fracture line or a spinal disc space. These bone plates generally include a plurality of holes through which fixation elements (e.g., bone screws, pins, etc.) are inserted to engage the bone. The holes of the plate may be of a such that each fixation element engages a hole in an orientation that is fixed or variable relative to the plate.

Angularly fixed bone plates and other supports are increasingly used in osteosynthesis applications. Plates with variable angle holes are particularly useful for treating fractures located near joints, or for anchoring screws in the spinal column. Shorter screws may often be inserted in a plate at preset angles without presenting problems. When longer screws are necessary, however, fixed, system-dependent orientations for the screws may be impractical or unwieldy.

### Summary of the Invention

The present invention is directed to a device for stabilizing portions of bone comprising a plate to be coupled to a target portion of bone including a first hole extending therethrough from a proximal surface to a distal, bone-facing, surface thereof and a first rigid member received within the first hole, the rigid member sized relative to the first hole to be rotatable therewithin about an axis substantially perpendicular to a proximal-distal axis of the first hole, the first rigid member including a first bore extending therethrough from a proximal opening to a distal opening, the first bore being sized and shaped to receive a first bone fixation element to be inserted through the plate into the target portion of bone in combination with a first deformable member coupling the first rigid member to the plate.

### Brief Description of the Drawings

Fig. 1 shows a top plan view of a bone plate according to an exemplary embodiment of the present invention;
Fig. 2 shows a longitudinal cross section of the bone plate of Fig. 1;
Fig. 3 shows a longitudinal cross section of the bone plate of Fig 1, with a flexible member deformed so that a bore extends therethrough at an angle;
Fig. 4 shows a cross-sectional perspective view of the bone plate of Fig. 1;
Fig. 5 shows a cross-sectional perspective view of a bone plate according to another embodiment of the present invention;
Fig. 6 shows a perspective view of a bone plate according to another embodiment of the present invention;
Fig. 7 shows a top plan view of the bone plate of Fig. 6;
Fig. 8 shows a cross-sectional perspective view of the bone plate of Fig. 6; and
Fig. 9 shows a top plan view of a bone plate according to a further embodiment of the present invention.

### Detailed Description

The present invention may be further understood with reference to the following description and the appended drawings, wherein like elements are referred to with the same reference numerals. The present invention relates to devices for treating fractures and, in particular, relates to an osteosynthetic implant such as a bone plate. Exemplary embodiments of the present invention provide a bone plate including a variable angle fixation element system, which allows a screw or other fixation element to engage a hole of the plate at a variable angle.

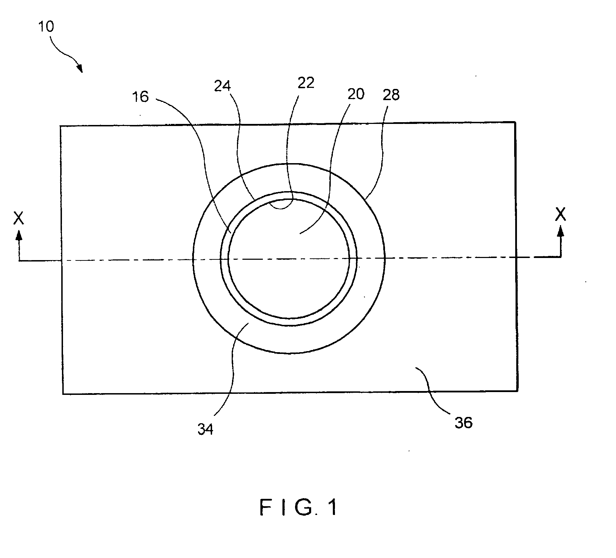

Figs. 1 - 4 show a bone plate 10 according to an exemplary embodiment of the present invention. The bone plate 10 comprises a proximal surface 12 which, when coupled to a bone in an operative position, faces away from the bone, a distal surface 14 which, in the operative position, faces the bone. A hole 15 extending through the plate 10 from the proximal surface 12 to the distal surface 14 includes a deformable member 18 coupled to a rigid member 16 received within the hole 15. The rigid member 16 includes a bore 20 extending therethrough and defines an inner surface 22 surrounding the bore 20 and an outer surface 24 facing a wall 28 of the hole 15. The member 18 allows the rigid member 16 to rotate relative to an axis A of the plate 10 (e.g., an axis substantially perpendicular to the plate 10) such that a fixation element may be inserted into the bore 20 of the rigid member 16 at a variable angle relative to the axis A.

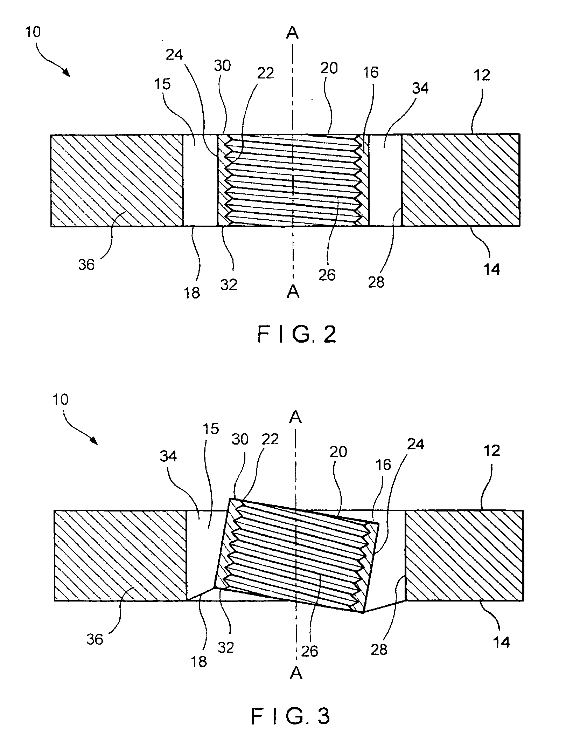

The rigid member 16 may have any shape corresponding to the hole 15. For example, the rigid member 16 may be substantially cylindrical with proximal and distal ends 30, 32, respectively, which may substantially align with the proximal and distal surfaces 12, 14, respectively when a longitudinal axis of the rigid member 16 is aligned with the axis A, as shown in Fig. 2. The bore 20 extends through the rigid member 16 and defines the inner surface 22, which is sized and shaped to receive a head of a bone fixation element such as a bone screw or pin. The inner surface 22 may include threading 26 to engage a corresponding threading of a head of a screw or pin to be inserted therethrough to lock the fixation element in the plate 10. It will be understood by those skilled in the art that the bore 20 may vary in size and shape depending on the type and size of fixation element, specifically, the size and shape of the head of the fixation element, to be used. For example, the bore 20 may be tapered to accommodate a fixation element having a similarly tapered head. In such a case, a diameter of the proximal opening 30 of the bore 20 is larger than a diameter of the distal opening 32 with a diameter of the bore 20 gradually diminishing as the distal end 32 is approached. It will also be understood by those of skill in the art that the outer surface 24 of the rigid member 16 may be non-cylindrical so long as the rigid member 16 is rotatably received in the hole 15 and the inner surface 22 may take on any shape so long as the bore 20 is adapted to receive a fixation element to be used therewith.

The rigid member 16 is connected to a portion 36 of the bone plate 10 surrounding the hole 15 by the deformable member 18, which connects the distal end 32 of the rigid member 16 to the portion 36 of the distal surface 14 surrounding the rigid member 16. The inner wall 28 of the surrounding portion 36 faces the outer surface 24 of the rigid member 16 such that a space 34 exists between the inner wall 28 and the outer surface 24 proximally of the member 18. In other words, the deformable member 18 surrounds the rigid member 16 at the distal end 32 thereof. The deformable member 18 may, for example, be formed of a thin, ductile material sufficiently malleable that the rigid member 16 may move at an angle relative to the axis A (rotate) in the space 34 while the distal end 32 remains connected to the surrounding portion 36 of the bone plate 10. As shown in Fig. 3, as the rigid member 16 rotates, the deformable member 18 deforms allowing one side of the rigid member 16 to move further from a portion of the wall 28 nearest thereto while the opposite side of the rigid member 16 moves further from a portion of the wall 28 nearest thereto. In a preferred embodiment, the rigid member 16 may rotate approximately 0° to 15° relative to the axis A.

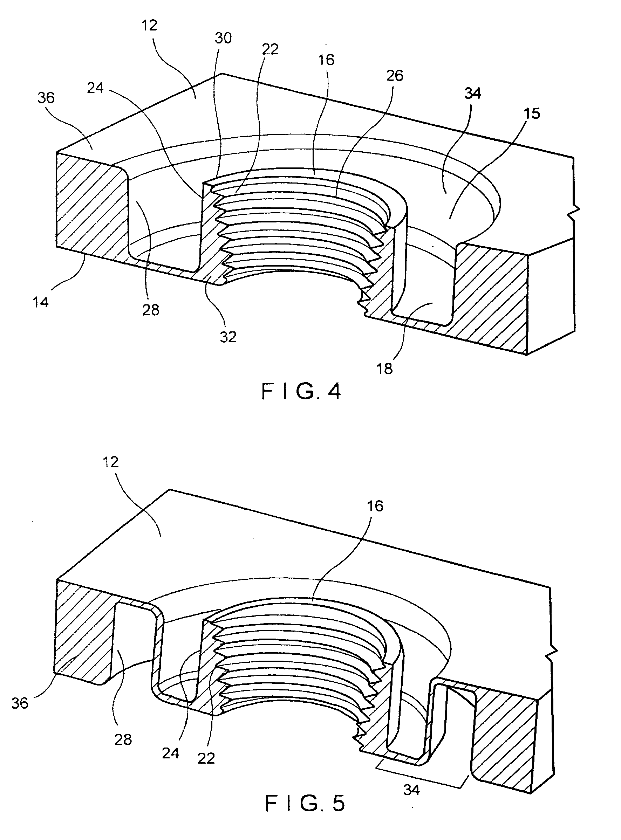

Those skilled in the art will understand that the deformable member 18 may be coupled to any portion of the rigid member 16 and any surface of the portion 36 of the plate 10 so long as the rigid member 16 is prevented from moving out of the hole 15 while being permitted to rotate relative to the plate 10 about an axis substantially perpendicular to an axis of the bore 20. For example, as shown in Fig. 5, the deformable member 18 may connect the distal end 32 of the rigid member 16 to the surrounding portion 36 at the proximal surface 12. Thus, the member 18 is rotatable within the space 34, which is created between the inner wall 28 of the surrounding portion 36 and the outer surface 24 of the rigid member 16.

The deformable member 18 is formed of a material that is plastically deformed as the rigid member 16 is rotated within the space 34 so that the rigid member 16 is maintained in a position to which it has been angled once the deformable member 18 has been deformed. Thus, the deformable member 18 may be pre-bent prior to a procedure with the rigid member 16 at a desired angle relative to the axis A using a drilling gauge. Alternatively, the deformable member 18 may be bent after a fixation element has been inserted through the rigid member 16 at an angle selected, for example, to provide compression to portions of the fractured bone. The deformable member 18 may be formed of glass metals, memory metals, grade 4 titanium, cold worked stainless steel and cobalt chrome alloy. It will be understood by those of skill in the art that the deformable member 18 may be formed of a variety of other materials, depending on the desired level of flexibility and/or rigidity.

It will be understood by those of skill in the art that the surrounding portion 36 may be rigid such that the deformable member 18 allows the rigid member 16 to move relative to the plate 10. The major portions of the plate 10, the surrounding portion 36 and the rigid member 16, may be formed of rigid bio-compatible materials such as stainless steel, titanium, titanium alloy and PEEK. It will also be understood by those of skill in the art that although the deformable member 18 is deformable while the surrounding portion 36 and the rigid member 16 are rigid, the plate 10 may be formed of a unitary construction such that no additional parts are necessary.

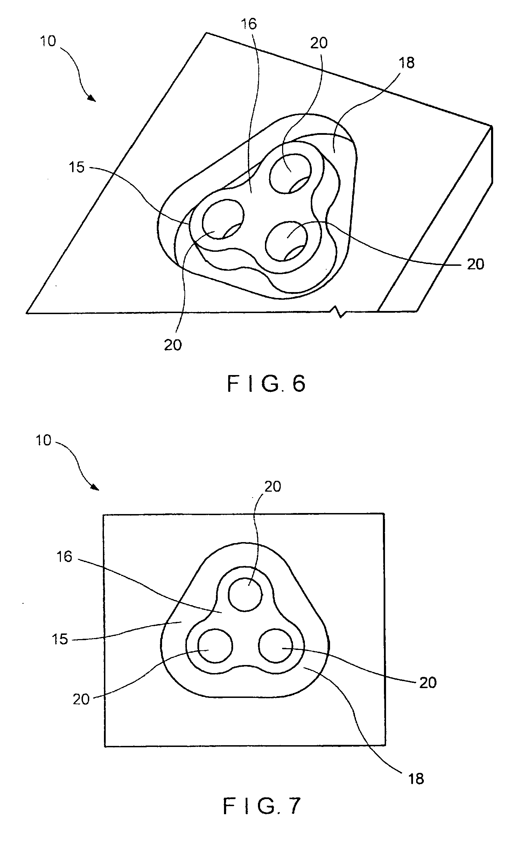

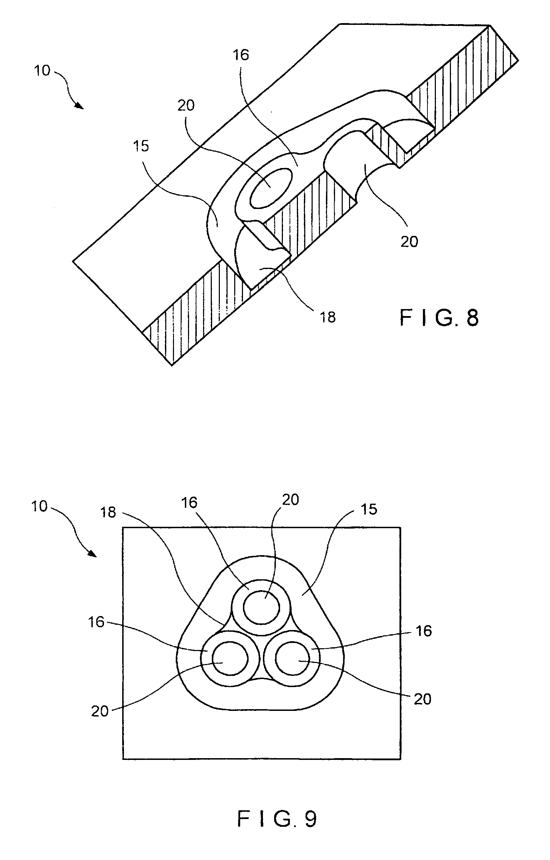

The rigid member 16 and the deformable member 18 may be incorporated into any of a variety of different types of bone plates 10, particularly plates for which it may be desired to adjust an angle at which a bone fixation element is inserted into a target portion of bone. It will also be understood by those of skill in the art that the bone plate 10 may include one or more holes 15 each including one or more rigid members 16 received therein. For example a single hole 15 may include one or a plurality of rigid members 16 and each rigid member 16 may include only a single bore 20 or a single rigid member 16 may include multiple bores 20. As would be understood by those skilled in the art, where a single rigid member 16 includes multiple bores 20, as shown in Figs. 6 - 8, these bores 20 may be parallel to one another or in any other angular orientation relative to one another. The rigidity of such a rigid member 16 allows a user to alter the orientation of an array of fixation elements inserted therethrough relative to the axis A but may not permit adjustment of the orientation of the fixation members of the array relative to one another. Where a plurality of rigid members 16 are received in a single hole 15, as shown in Fig. 9, the deformable member 18 is preferably sufficiently flexible to permit the bores 20 of each of the rigid members 16 to be individually aligned with corresponding holes via which they are to be inserted into target portions of the bone. That is, in such an arrangement, each of the rigid members 16 is independently adjustable relative to the other rigid members 16 in the same hole 15. Thus, such an arrangement may be more suitably coupled with a deformable member 18 sufficiently flexible that each rigid member 16 may be moved to any of a variety of angles and thereafter returned to an original configuration. Furthermore, as would be understood by those skilled in the art, the plate 10 may comprise any number and arrangement of holes 15 with corresponding rigid members 16 as well as any desired number of conventional fixation element receiving holes (e.g., fixed angle or variable angle holes, locking and non-locking holes, so called combination holes, etc.) as dictated by the intended use of the plate 10.

In an alternate embodiment, the deformable member 18 may be formed of a mesh-like structure between the outer surface 24 of the rigid member 16 and the inner wall 28 of the surrounding portion 36 of the plate 10. The mesh-like structure may be sufficiently flexible such that the rigid member 16 may rotate within the hole 15. In a further alternate embodiment, the rigid member 16 may also be replaced by the mesh-like structure such the hole 15 may be filled with the mesh-like structure. The mesh-like structure may be penetratable such that a fixation element may be inserted through the mesh-like structure without requiring a hole to be pre-drilled. The mesh-like structure may be a soft grid that may be filled with any kind of plastic material to prevent ingrowing and/or prevent loose parts and/or debris to migrate.

It will be apparent to those skilled in the art that various modifications and variations can be made in the structure and methodology of the present invention, without departing from the scope of the invention. Thus, it is intended that the present invention cover the modifications and variations of this invention provided that they come within the scope of the appended claims and their equivalents.

## Claims

1. A device for stabilizing portions of bone, comprising:
a plate (10) to be coupled to a target portion of bone including a first hole (15) extending therethrough from a proximal surface (12) to a distal, bone-facing, surface (14) thereof;
a first rigid member (16) received within the first hole (15), the first rigid member (16) sized relative to the first hole (15) to be rotatable therewithin about an axis substantially perpendicular to a proximal-distal axis of the first hole (15), the first rigid member (16) including a first bore (20) extending therethrough from a proximal opening to a distal opening, the first bore being sized and shaped to receive a first bone fixation element to be inserted through the plate into the target portion of bone;
and
a deformable member (18) coupling the first rigid (16) member to the plate (10),
**characterized in that**
the deformable member (18) surrounds the first rigid member (16) at a distal end (32) thereof; and wherein
the deformable member (18) is formed of a material that is plastically deformed as the first rigid member (16) is rotated relative to the plate (10).

2. The device of claim 1, wherein the first hole includes a second rigid member, the second rigid member including a second bore extending therethrough.

3. The device of claim 1, wherein the first rigid member includes a second bore.

4. The device of claim 3, wherein the second bore is parallel to the first bore.

5. The device of claim 3, wherein the second bore is angled relative to the first bore.

6. The device of claim 1, wherein the plate includes a second bore and a second rigid member.

7. The device of claim 1, wherein the first bore is threaded to lockingly receive a correspondingly threaded head of the first fixation element.

8. The device of claim 7, wherein the first bore is tapered to correspond to a shape of a tapered head of the first fixation element inserted thereinto.

9. The device of claim 2, wherein the first hole is adapted to receive a bone screw.

10. The device of claim 2, wherein the first hole is adapted to receive a pin.

11. The device of claim 1, wherein the first rigid member and the first hole are substantially cylindrical.

12. The device of claim 1, wherein a proximal end of the rigid member substantially aligns with a proximal surface of the implant and a distal end of the rigid member substantially aligns with a distal surface of the implant when the membrane is not deformed.

13. The device of claim 1, wherein a proximal end of the rigid member substantially aligns with a proximal surface of the implant and a distal end of the rigid member substantially aligns with a distal surface of the implant when a longitudinal axis of the first bore aligns with a longitudinal axis of the first hole.

14. The device of claim 1, wherein the plate is formed of one of stainless steel, titanium, titanium alloy and PEEK.

15. The device of claim 1, wherein the rigid member is formed of one of stainless steel, titanium, titanium alloy and PEEK.

## Patentansprüche

1. Vorrichtung zur Stabilisierung von Knochenabschnitten umfassend:
eine Platte (10) zur Verbindung mit einem Zielabschnitt eines Knochens umfassend ein erstes Loch (15), welches sich von einer proximalen Oberfläche (12) zu einer distalen,
einem Knochen zugewandten Oberfläche (14) der Platte erstreckt;
ein erstes rigides Teil (16), welches im ersten Loch (15) aufgenommen ist, wobei das erste rigide Teil (16) relativ zum ersten Loch (15) so bemessen ist, dass es darin um eine zu einer proximal-distalen Achse des ersten Lochs (15) im Wesentlichen senkrechte Achse rotierbar ist, wobei das erste rigide Teil (16) eine erste Bohrung (20) umfasst, welche sich durch das erste Teil von einer proximalen Öffnung zu einer distalen Öffnung erstreckt, wobei die erste Bohrung zur Aufnahme eines ersten Knochenfixationselements bemessen und geformt ist, welches durch die Platte in einen Zielabschnitt eines Knochens einzuführen ist; und
ein deformierbares Teil (18), welches das erste rigide Teil (16) mit der Platte (10) verbindet,
**dadurch gekennzeichnet, dass**
das deformierbare Teil (18) das erste rigide Teil (16) an einem distalen Ende (32) davon umschliesst; und wobei
das deformierbare Teil (18) aus einem Material geformt ist, das plastisch verformt wird, wenn das erste rigide Teil (16) relativ zur Platte (10) rotiert wird.

2. Vorrichtung nach Anspruch 1, **dadurch gekennzeichnet, dass** das erste Loch ein zweites rigides Teil umfasst, wobei das zweite rigide Teil eine zweite Bohrung umfasst, welche sich durch dasselbe erstreckt.

3. Vorrichtung nach Anspruch 1, **dadurch gekennzeichnet, dass** das erste rigide Teil eine zweite Bohrung umfasst.

4. Vorrichtung nach Anspruch 3, **dadurch gekennzeichnet, dass** die zweite Bohrung parallel zur ersten Bohrung ist.

5. Vorrichtung nach Anspruch 3, **dadurch gekennzeichnet, dass** die zweite Bohrung relativ zur ersten Bohrung abgewinkelt ist.

6. Vorrichtung nach Anspruch 1, **dadurch gekennzeichnet, dass** die Platte eine zweite Bohrung und ein zweitens rigides Teil umfasst.

7. Vorrichtung nach Anspruch 1, **dadurch gekennzeichnet, dass** die erste Bohrung gewindet ist, um einen entsprechend gewindeten Kopf des ersten Fixationselements verriegelbar aufzunehmen.

8. Vorrichtung nach Anspruch 7, **dadurch gekennzeichnet, dass** sich die erste Bohrung verjüngt, um einer Form eines sich verjüngenden Kopfs des darin eingeführten ersten Fixationselements zu entsprechen.

9. Vorrichtung nach Anspruch 2, **dadurch gekennzeichnet, dass** das erste Loch zur Aufnahme einer Knochenschraube adaptiert ist.

10. Vorrichtung nach Anspruch 2, **dadurch gekennzeichnet, dass** das erste Loch zur Aufnahme eines Pins adaptiert ist.

11. Vorrichtung nach Anspruch 1, **dadurch gekennzeichnet, dass** das erste rigide Teil und das erste Loch im Wesentlichen zylindrisch sind.

12. Vorrichtung nach Anspruch 1, **dadurch gekennzeichnet, dass** ein proximales Ende des rigiden Teils im Wesentlichen zu einer proximalen Oberfläche des Implantats ausgerichtet ist und ein distales Ende des rigiden Teils im Wesentlichen zu einer distalen Oberfläche des Implantats ausgerichtet ist, wenn die Membran nicht verformt ist.

13. Vorrichtung nach Anspruch 1, **dadurch gekennzeichnet, dass** ein proximales Ende des rigiden Teils im Wesentlichen mit einer proximalen Oberfläche des Implantats ausgerichtet ist und ein distales Ende des rigiden Teils im Wesentlichen zu einer distalen Oberfläche des Implantats ausgerichtet ist, wenn eine Längsachse der ersten Bohrung mit eine Längsachse des ersten Lochs ausgerichtet ist.

14. Vorrichtung nach Anspruch 1, **dadurch gekennzeichnet, dass** die Platte aus Edelstahl, Titan, einer Titan-Legierung oder PEEK hergestellt ist.

15. Die Vorrichtung nach Anspruch 1, **dadurch gekennzeichnet, dass** das rigide Teil aus Edelstahl, Titan, einer Titan-Legierung oder PEEK hergestellt ist.

## Revendications

1. Système pour stabiliser des parties d'un os, comprenant :
une plaque (10) à coupler à une partie cible de l'os comportant un premier trou (15) s'étendant à travers elle, à partir de sa surface proximale (12) jusqu'à sa surface distale, opposée à l'os (14) ;
un premier élément rigide (16) reçu à l'intérieur du premier trou (15), le premier élément rigide (16) étant dimensionné par rapport au premier trou (15) afin de pouvoir tourner à l'intérieur de celui-ci autour d'un axe essentiellement perpendiculaire à un axe proximal-distal du premier trou (15),
le premier élément rigide (16) comportant un premier alésage (20) s'étendant à travers lui à partir d'une ouverture proximale jusqu'à une ouverture distale,
le premier alésage étant dimensionné et configuré pour recevoir un premier élément de fixation à l'os à insérer à travers la plaque dans la partie cible de l'os ; et
un élément déformable (18) couplant le premier élément rigide (16) à la plaque (10),
**caractérisé en ce que**
l'élément déformable (18) entoure le premier élément rigide (16) au niveau de son extrémité distale (32) ; et dans lequel
l'élément déformable (18) est constitué d'un matériau qui est plastiquement déformé lorsque le premier élément rigide (16) est tourné par rapport à la plaque (10).

2. Système selon la revendication 1, dans lequel le premier trou comprend un second élément rigide, le second élément rigide comportant un second alésage s'étendant à travers lui.

3. Système selon la revendication 1, dans lequel le premier élément rigide comporte un second alésage.

4. Système selon la revendication 3, dans lequel le second alésage est parallèle au premier alésage.

5. Système selon la revendication 3, dans lequel le second alésage est incliné par rapport au premier alésage.

6. Système selon la revendication 1, dans lequel la plaque comporte un second alésage et un second élément rigide.

7. Système selon la revendication 1, dans lequel le premier alésage est taraudé pour recevoir de façon à la bloquer une tête filetée de façon correspondante du premier élément de fixation.

8. Système selon la revendication 7, dans lequel le premier alésage est conique de façon à correspondre à une configuration d'une tête conique du premier élément de fixation inséré en lui.

9. Système selon la revendication 2, dans lequel le premier trou est adapté pour recevoir une vis destinée à l'os.

10. Système selon la revendication 2, dans lequel le premier trou est adapté pour recevoir une broche.

11. Système selon la revendication 1, dans lequel le premier élément rigide et le premier trou sont essentiellement cylindriques.

12. Système selon la revendication 1, dans lequel une extrémité proximale de l'élément rigide s'aligne essentiellement avec une surface proximale de l'implant et dans lequel une extrémité distale de l'élément rigide s'aligne essentiellement avec une surface distale de l'implant lorsque la membrane n'est pas déformée.

13. Système selon la revendication 1, dans lequel une extrémité proximale de l'élément rigide s'aligne essentiellement avec une surface proximale de l'implant et dans lequel une extrémité distale de l'élément rigide s'aligne essentiellement avec une surface distale de l'implant lorsqu'un axe longitudinal du premier alésage s'aligne avec un axe longitudinal du premier trou.

14. Système selon la revendication 1, dans lequel la plaque est constituée de l'un parmi acier inoxydable, titane, alliage de titane et PEEK.

15. Système selon la revendication 1, dans lequel l'élément rigide est constitué de l'un parmi acier inoxydable, titane, alliage de titane et PEEK.
